# EUROPÄISCHE PATENTSCHRIFT

(11) **EP 1 201 709 B1**
(45) Veröffentlichungstag und Bekanntmachung des Hinweises auf die Patenterteilung: **09.04.2003**
(21) Anmeldenummer: 00811097.5
(22) Anmeldetag: 20.11.2000
(51) Int. Cl.: C08L 1/02, C08L 3/02, A61K 9/20, A61K 47/36

(54) **Coprozessiertes Sprengmittelgranulat**
Coprocessed granules of disintegrating agents
Granules d'agents désintégrants co-travaillés

(30) Priorität: 24.10.2000 CH 20002078
(43) Veröffentlichungstag der Anmeldung: 02.05.2002
(73) Patentinhaber: fit GmbH, 02788 Hirschfelde (DE)
(72) Erfinder: Streb, Dr. Martin, 4102 Binningen (CH); Bauer, Prof. Dr. Kurt, 79112 Freiburg (DE)
(74) Vertreter: Sommer, Peter

(56) Entgegenhaltungen:
- EP-A- 1 006 148
- WO-A-89/02266
- WO-A-99/09959

## Beschreibung

Die Erfindung betrifft ein coprozessiertes Sprengmittelgranulat für in Flüssigkeit aufzulösende Festkörper, welches sich einerseits durch leichte plastische Verformbarkeit auszeichnet, das Basischarakteristikum für Trockenbindemittel und einer guten Tablettierbarkeit, und andererseits durch eine ausserordentlich schnelle und starke partikuläre Quellung beim Einbringen in Wasser oder wasserhaltige Flüssigkeiten. Da diese Eigenschaften mit einer Einzelsubstanz offenbar nicht erreichbar sind, werden sie durch die innige Vermischung und Coprozessierung von geeigneten natürlichen oder synthetischen Hilfsstoffen mit mehr oder weniger ausgeprägten Wassertransport- und Quelleigenschaften angestrebt. Als geeignete Hilfsstoffe für den beabsichtigten Zweck werden unlösliche, aber größere Mengen Wasser aufnehmende und stark quellende natürliche oder synthetische Hilfsstoffe eingesetzt, beispielsweise die Polysaccharide Cellulose und Stärke oder ein vemetztes Polyvinylpyrrolidon, sowie unlösliche Carboxymethylstärken und unlösliche Carboxymethylcellulosen.

Das vemetzte Polyvinylpyrrolidon, sowie die unlöslichen Polysaccharide Carboxymethylstärke und Carboxymethylcellulose, sind sogenannte Sprengmittel und zeigen infolge ihrer extremen Hydrophilität ein starkes Quellverhalten und auf Grund ihrer Unlöslichkeit eine starke Sprengwirkung. Im Gegensatz dazu, zeigen diese jedoch kaum plastische Trockenbindemittel- oder Tablettiereigenschaften und auch keine Wassertransportfähigkeiten, da sie zum Quellen sehr viel Wasser aufnehmen, aber nicht abgeben, sondern fest binden. Cellulosepulver dagegen ist ein ausserordentlich plastisches Trockenbindemittel, es ist ausserdem fähig unter mässiger Quellung viel Wasser aufzunehmen und relativ leicht wieder abzugeben. Deshalb kann Cellulose als eine Art Dochtsubstanz wirken, die rasch und viel Wasser in das Coprozessat oder in eine Formulierung aufsaugt, dieses Wasser aber sofort wieder an ein Quellmittel abgibt, damit dieses optimal quellen und die Formulierung sprengen kann. Gekörnte native Stärke liegt mit ihren Eigenschaften zwischen Dochtsubstanz und Quellmittel. Cellulosen und Stärken sind Polysaccharide. Während gekörnte native Stärken globuläre Mehrschichtstrukturen zeigen, haben die Cellulosen vorwiegend fibrilläre Strukturen. Die kugelförmigen Stärkekörner quellen unter Wasseraufnahme partikulär, geben das Wasser, je nach dem wie tief es in den Schichten liegt, auch mehr oder weniger gut ab, sie sind aber elastisch und nicht plastisch und besitzen demzufolge keinerlei Bindeeigenschaften. Aus reinen Stärken kann man wegen der vorherrschenden Elastizität keine Tabletten herstellen. Die plastischen Strukturen der Cellulosen lassen sich relativ leicht plastisch verformen, dabei verhaken sich die einzelnen Partikel zu sogenannten formschlüssigen Verbindungen und darauf begründet sich ihre hervorragende Eignung als Trockenbindemittel. Cellulosen können auch relativ viel Wasser aufnehmen und weitergeben. Durch diese Dochtwirkung wird Feuchtigkeit an- und aufgesaugt und zum Sprengmittel weitergeleitet, um dieses mit der, zum optimalen Quellen, notwendigen und ausreichenden Flüssigkeitsmenge zu versorgen. Werden Cellulosen zu Tabletten verpresst und diese in eine wässrige Umgebung eingebracht, dann quellen diese Tabletten auf, aber sie zerfallen nicht, da die einzelnen Cellulosepartikel zu stark ineinander verhakt sind. Wird ein Gemisch aus Stärken und Cellulosen tablettiert oder kompaktiert, dann entstehen durch die Bindeeigenschaften der Cellulosen und den Quellund Dochteigenschaften von Cellulosen und Stärken gut zusammenhaltende, in Wasser quellende und zerfallende Tabletten oder Granulate. Die Quelleigenschaften sind jedoch bei Weitem noch nicht optimal, deshalb ist der Zusatz einer dritten, sehr stark quellenden Komponente erforderlich.

In der EP 1 006 148 A1 sind gut tablettierbare, coprozessierte Polysaccharidprodukte aus Natrium-Carboxymethylstärke und mikrokristalliner oder pulverisierter Cellulose zur Verwendung als Tablettensprengmittel offenbart.

In der WO 89/02266 A sind pharmazeutische Granulate angegeben, die Ibuprofen und ein pharmazeutisch akzeptables Sprengmittel aus Carboxymethylcellulose enthalten, zu denen evtl. gelatisierte Stärke und mikrokristalline Cellulose, jeweils als Bindemittel und Sprengmittel zugefügt werden kann. Auch können Polyvinylpyrrolidon sowie Stärke beigefügt werden.

Die WO 99/09959 A schlägt Tabletten aus Methylcellulose vor, die schnell zerfallen und zu der man mikrokristalline Cellulose sowie Stärke als Füllmittel hinzufügen kann. Desweiteren sind Bindemittel, wie Polyvinylpyrrolidon, und Sprengmittel wie Carboxymethylcellulose erwähnt. Als Tablettensprengmittel (Zerfallsbeschleuniger, Zerfallhilfsmittel) werden Hilfsstoffe bezeichnet, die für den raschen Zerfall von Tabletten in Wasser oder Magensaft, und für die Freisetzung der Wirkstoffe in fein dispergierter, gut resorbierbarer Form sorgen. Je nach Wirkungsmechanismus handelt es sich um Substanzen, die die Porosität der Komprimate erhöhen und ein großes Adsorptionsvermögen bei nicht zu starken oder länger anhaltenden Bindeeigenschaften für Wasser besitzen. Beispiele für bekannte Tablettensprengmittel sind Stärken, derivatisierte Stärke, wie unlösliche Carboxymethylstärke, unlösliche Cellulosederivate, wie z.B. Carboxymethylcellulose, Alginsäuren, Dextrane und vernetzte Polyvinylpyrrolidone. Auch gasentwickelnde Substanzgemische, z.B. Natriumhydrogencarbonat und Zitronen- oder Weinsäure (Brausemischungen) können zu den Zerfallsbeschleunigern gerechnet werden. Diese besitzen jedoch einen völlig anderen Zerfallsmechanismus.

Die im Stand der Technik bekannten Tablettensprengmittel, vor allem die Spreng- oder Quellmittel, wie beispielsweise die unlöslichen Polysaccharide Carboxymethylstärke und Carboxymethylcellulose, sowie vernetztes Polyvinylpyrrolidon, sind noch mit folgenden Nachteilen behaftet:

Nicht alle Tablettensprengmittel, insbesondere die Spreng- und Quellmittel, sind ausreichend plastisch, sondern vielmehr elastisch und deshalb schlecht plastisch verformbar bzw. tablettier- oder kompaktierbar. Insbesondere Stärken zählen zu den elastischen Quellmitteln.

Celluloseprodukte sind dagegen mehr plastisch und deshalb gut komprimierbar. Als gut plastisch verformbares Trockenbindemittel festigt die Cellulose den Zusammenhalt von festen Formen, wie Granulaten oder Tabletten. Die Cellulosen wirken in Kombination mit Quell- oder Sprengmitteln vorwiegend als Dochtsubstanz, die ausreichende Wassermengen mit ausreichender Geschwindigkeit ansaugen und relativ leicht wieder abgeben, damit die sogenannten Quell- oder Sprengmittel ihren Quelleffekt voll entfalten können. Diese können stark quellen, wenn ihnen ausreichende Wassermengen zugeführt werden. Reine Cellulosetabletten nehmen zwar grössere Wassermengen auf und quellen auch gut, ohne jedoch dabei zu zerfallen. Durch eine ausgewogene Mischung von bindenden Dochtmitteln, quellenden Dochtmitteln und reinen Quellmitteln könnten die Vorteile der guten Tablettierbarkeit, der starken Quellung und der schnellen Zerfallbarkeit vereinigt und optimiert werden. Allerdings darf keine der erforderlichen Eigenschaften dominieren.

Aufgabe der vorliegenden Erfindung ist die Schaffung eines Sprengmittelgranulats welches die vorangehend aufgeführten Nachteile der bisher bekannten Sprengmittel nicht aufweist, d.h. das Finden einer Dreiermischung aus bindenden Dochtmitteln, quellenden Dochtmitteln und reinen Quellmitteln, und deren Eigenschaft derart aufeinander abzustimmen, dass schliesslich auch gegenläufige Eigenschaften, wie Bindekräfte und Zerfall, gut zusammenwirken.

Diese Aufgabe wird mittels eines coprozessierten Sprengmittelgranulats gemäss Anspruch 1 gelöst.

Zweckmässige Ausführungsformen des erfindungsgemässen Sprengmittelgranulats sind Gegenstand der abhängigen Ansprüche 2 bis 6.

Gegenstand der vorliegenden Erfindung ist ferner ein Verfahren zur Herstellung des erfindungsgemässen Sprengmittelgranulats nach Anspruch 7. Vorteilhafte Ausführungsformen dieses Verfahrens sind Gegenstand der abhängigen Ansprüche 8 und 9.

Überraschend wurde gefunden, dass sich durch feuchte oder trockene Verdichtung oder Granulierung des Ausgangspulvergemisches ein erheblich gesteigerter optimaler Gesamteffekt erzielen lässt.

Es ergeben sich auch gute Zerfallseigenschaften, wenn die drei genannten Komponenten, wie es üblicherweise geschieht, einzeln in Tabletten- oder Granulatformulierungen eingearbeitet werden. Der Effekt wird jedoch erfindungsgemäss erheblich verbessert, wenn die drei Komponenten durch eine geeignete Coprozessierung, z.B. durch feuchte oder trockene Granulierung und/oder Kompaktierung, sehr eng und dicht miteinander verbunden werden, bevor sie in eine Formulierung, beispielsweise in Tabletten eingearbeitet werden. Offensichtlich wird die Docht- und Quellwirkung durch die enge Vereinigung vorteilhaft optimiert.

Die neuen Produkte sind sowohl durch eine sehr gute Tablettierbarkeit als auch durch eine hervorragende Zerfallbarkeit charakterisiert. Sie können daher mit Erfolg als Tablettensprengmittel (Zerfallsbeschleuniger, Zerfallhilfsmittel) eingesetzt werden.

Das erfindungsgemässe, durch Coprozessierung von Sprengmittel mit Cellulosepulver oder mikrokristalliner Cellulose und mit nativer Stärke erhaltene Granulat ist zweckmässigerweise durch folgende Kenndaten charakterisiert:

Bei den gemahlenen, coprozessierten Zubereitungen sollen die Schüttdichten der Produkte mit Cellulosepulver bei 0,120-0,600 g/ml liegen, vorzugsweise bei 0,250-0,500 g/ml. Bei den Coprodukten mit mikrokristalliner Cellulose liegen die Schüttdichten bei 0,300-0,750 g/ml, vorzugsweise bei 0,350-0,600 g/ml. Die Schüttdichte des erfindungsgemässen Sprengmittelgranulats ist höher als die Schüttdichte des Ausgangspulvergemisches.

Insbesondere ist das Produkt durch einen Restfeuchtegehalt von 1,5 bis 15%, vorzugsweise von weniger als 10%, beispielsweise 5 bis 9%, charakterisiert.

Der Gehalt der einzelnen Komponenten kann innerhalb weiter Grenzen variieren, beispielsweise 20 bis 80 Gew.-% Cellulose, 10 bis 40 Gew.-% native Stärke, sowie 10 bis 40 Gew.-% eines in Wasser und in wässrigen Flüssigkeiten unlöslichen Sprengmittels.

Auch die Korngrösse des erfindungsgemässen Sprengmittelgranulates kann innerhalb weiter Grenzen variieren, wie beispielsweise von 10 bis 3000 µm, vorzugsweise von 200 bis 2000 µm.

Das erfindungsgemässe Sprengmittelgranulat kann durch Coprozessierung bzw. gemeinsame Behandlung von Sprengmittel mit pulverisierter oder mikrokristalliner Cellulose und nativer Stärke durch feuchte oder trockene Aggregierung unter Druck erhalten werden. Unter dem Begriff "Coprozessierung" wird hier eine trockene Kompaktierung/Aggregierung, z.B. zwischen gegenläufigen Kompaktierwalzen, oder eine feuchte Kompaktierung/Aggregierung nach Zusatz von Wasser, durch Kneten oder Pressen von feuchtplastischen Massen durch ein Sieb, eine Lochscheibe oder über einen Extruder, und abschliessendem Trocknen, verstanden.

Durch die Erfindung wird weiterhin ein Verfahren zur Herstellung des oben beschriebenen Sprengmittelgranulates bereitgestellt, welches die Coprozessierung von Sprengmittel mit mikrokristalliner oder pulverisierter Cellulose und nativer Stärke umfasst.

Eine Alternative des erfindungsgemässen Verfahrens ist durch folgende Stufen charakterisiert:
(a) Suspendieren von feuchter bzw. nicht getrockneter, nicht verhornter mikrokristalliner oder pulverisierter Cellulose in einem, vorzugsweise heissen, wässrigen Medium zu einer homogenen Dispersion;
(b) Vermengen der in Stufe (a) erhaltenen Suspension oder pastenförmigen, vorverdichteten Massen nach Abkühlen unter 60°C mit nativer Stärke und Polymer;
(c) gegebenenfalls erfolgendes Vortrocknen;
(d) Aggregieren unter Druck, insbesondere verdichtendes Passieren, vorzugsweise Pressen der in Stufe (b) bzw. (c) erhaltenen und vorverdichteten feuchtplastischen Masse durch ein Sieb, eine Lochscheibe oder einen Extruder;
(e) Trocknen der in Stufe (d) erhaltenen aggregierten Masse bei einer Temperatur von 20 bis 90°C; und
(f) Vermahlen des so erhaltenen Produkts auf eine mittlere Komqrösse von 10 bis 3000 µm, vorzugsweise von 200 bis 2000 µm.

In der ersten Stufe dieses Verfahrens wird vorzugsweise ein nicht getrocknetes mikrokristallines Celluloseprodukt oder pulverisierte Cellulose in einem wässrigen Medium zu einer homogenen Dispersion suspendiert. Die pulverförmige Cellulose wird beispielsweise in an sich bekannter Weise aus Zellstoff oder faseriger Rohcellulose hergestellt. Die mikrokristalline Cellulose wird aus Zellstoffpulpe durch Erhitzen mit einer 3- bis 5%-igen starken Mineralsäure, beispielsweise Salz- oder Schwefelsäure, hergestellt. Für die Zwecke der Erfindung besonders geeignete, nicht getrocknete Celluloseprodukte sind praktisch Zwischen- oder Vorprodukte der Herstellung von reiner Pulvercellulose oder mikrokristalliner Cellulose.

Die oben erwähnte Herstellung von mikrokristalliner Cellulose aus Zellstoff oder von Pulvercellulose wird beispielsweise von B. Philipp und H.H. Steege in "Wissenschaft und Fortschritt" 25 (3), 126-131 (1975) beschrieben.

Unter "nicht getrocknet" werden Cellulosevorprodukte verstanden, die aus dem Herstellungsprozess, nach der Reinigung, aber vor der Endtrocknung gewonnen werden, z.B. bei der oben beschriebenen Behandlung von Zellstoffpulpe mit Mineralsäure nach Entfernen der Säure und der erforderlichen Reinigung der hierbei entstandenen mikrokristallinen Cellulose. Das Cellulose : Wasser-Verhältnis kann 1,0:0,15 Teile bis 1,0:30,0 Teile, vorzugsweise 1,0:1,0 Teile bis 1,0:10,0 Teile betragen. Bei einer Trocknung des Produkts zu einem Feuchtigkeitsgehalt von weniger als 1,5 Teile Wasser pro 10,0 Teile Cellulose kann es vorkommen, daß die Oberfläche der Cellulose zu trocken wird, und dass das Produkt in unerwünschter Weise "verhornt". In solchen Fällen sind die Polysaccharidprodukte nicht mehr optimal für die oben angegebenen Zwecke geeignet, da verhomte Bereiche nur mehr stark eingeschränkt hydratisieren können. Andererseits führen zu grosse Flüssigkeits- bzw. Wassermengen dazu, daß die Produkte nach der Zugabe von nativer Stärke und unlöslichem Polymer nicht ausreichend feuchtplastisch werden, sondern zu flüssig sind, so daß eine Obergrenze von 30,0 Teile, vorzugsweise 25,0 Teile Wasser pro 1,0 Teil Cellulose bevorzugt wird. Außerdem ist das Trocknen von hohen Feuchtigkeitsanteilen nicht wirtschaftlich. Relativ hohe Mengen von Wasser sind zu einer ausreichenden Verdichtung erforderlich, aber zu hohe Mengen können entsprechend den in Stufe (b) verwendeten Mengen an nativer Stärke und Polymer, je nach Vermahlungs- und/oder Polymerisierungsgrad, die Gemische dann zu feucht werden lassen, so dass sie nicht mehr feuchtplastisch verformbar bzw. aggregierbar sind, und nur noch langwierig unter Schwierigkeiten getrocknet werden können.

Somit wird in der ersten Stufe dieses Verfahrens die oben beschriebene nicht verhornte Cellulose in einem wässrigen, zur besseren Hydratisierung vorzugsweise warmen bis heissen Medium zu einer homogenen Dispersion suspendiert. Als wässriges Medium kann beispielsweise Wasser, wie Leitungswasser, verwendet werden, das gegebenenfalls bis zum Sieden aufgeheitzt werden kann. Die Temperatur beim Suspendierungsvorgang beträgt 40 bis 100°C, vorzugsweise 80 bis 100°C. Die Suspendierung kann in an sich bekannter Weise in einer geeigneten Vorrichtung, wie einem Schnellmischer oder Dispergator, verwendet werden. Geeignete Vorrichtungen sind im Handel erhältlich und werden beispielsweise in Bauer, Frömming, Führer: Pharmazeutische Technologie, G. Fischer Verlag, Stuttgart, Jena, 5. Auflage, 1997, Seite 135 beschrieben. Bei dieser Befeuchtung, d.h. bei diesem Suspensionsvorgang, kann gegebenenfalls schon eine Vorverdichtung erzielt werden, z.B. durch stärkere Hydratation bei Verwendung von heissem Wasser. Weiterhin kann durch Kneten, beispielsweise im Z-Kneter mit kräftigen Mischarmen, im Gegensatz zu einfach gerührten bzw. gemischten Massen, eine stärkere Verdichtung oder Vorverdichtung erreicht werden. Die so erhaltene Dispersion hat beispielsweise einen Feststoffgehalt von 10 bis 90 Gew.-%, vorzugsweise 20 bis 60 Gew.-%.

In der nächsten Stufe (b) dieses Verfahrens wird die in Stufe (a) erhaltene Suspension nach Abkühlung unter 60°C mit nativer Stärke und mit unlöslichem Polymer vermengt Je nach verwendetem Polymer bzw. Sprengmittel können hierbei alle beliebigen im Handel erhältlichen Natrium-Carboxymethylstärke-, Carboxymethylcellulose- und vernetzte Polyvinylpyrrolidonsorten verwendet werden.

Die Menge des in dieser Stufe zugegebenen Sprengmittels wird so bemessen, daß die als Endprodukt erhaltenen Granulate 10 bis 40 Gew.-% Sprengmittel enthalten.

Die Vermengung der drei Ausgangsprodukte kann vorzugsweise durch Verkneten geschehen. Geeignete Kneter werden beispielsweise in Bauer, Frömming, Führer: Pharmazeutische Technologie, G. Fischer Verlag, Stuttgart, Jena, 5. Auflage, 1997, Seite 134 -135 beschrieben. Die Temperatur bei der Vermengung bzw. Verknetung beträgt 20 bis 60°C, vorzugsweise 20 bis 40°C. Vorzugsweise erfolgt die Verknetung bei Raumtemperatur, damit die Stärke und das Sprengmittel nicht nachteilig beeinflußt werden. Eine Verknetung kann daneben auch grundsätzlich vorgenommen werden, um eine Verdichtung zu erzielen.

In der nächsten Stufe (c) dieses Verfahrens wird das in Stufe (b) erhaltene Gemisch gegebenenfalls zu einem Restfeuchtegehalt von 1,5 bis 15 Gew.-%, vorzugsweise 5 bis 9 Gew.-%, vorgetrocknet. Als Trockner können übliche Trocknungsvorrichtungen, wie Trockenschränke und Wirbelschichttrockner eingesetzt werden. Geeignete Trockner werden beispielsweise in Bauer, Frömming, Führer: Pharmazeutische Technologie, G. Fischer Verlag, Stuttgart, Jena, 5. Auflage, 1997, Seiten 123-130 beschrieben. Bevorzugt wird die Vortrocknung bei Temperaturen von 30 bis 70°C. insbesondere 50 bis 60°C, durchgeführt.

In der nächsten Stufe (d) dieses Verfahrens wird das in Stufe (b) bzw. (c) erhaltene feuchtplastische, durch das Befeuchten bereits vorverdichtete Produkt durch ein Sieb geschlagen bzw. gepresst und dadurch möglichst weitgehend verdichtet. Als Siebe kommen beispielsweise übliche Siebe, die beispielsweise in Bauer, Frömming, Führer: Pharmazeutische Technologie, G. Fischer Verlag, Stuttgart, Jena, 5. Auflage, 1997, Seite 108 (Siebe), Seite 308 (Extrudergranulierung) beschrieben werden, in Betracht. Geeignete Siebe haben eine Maschenweite von 0,5 mm bis 5 mm, vorzugsweise 1-2 mm. Im übrigen erfolgt das Durchpressen bzw. -schlagen des Produktes durch Siebe bzw. durch Extruder in üblicher Weise und bei üblichen Bedingungen, wie Normaltemperatur. Das Sieben erfolgt bei Normaldruck oder gegebenenfalls unter Anwendung von Überdruck.

In der nächsten Stufe (e) dieses erfindungsgemässen Verfahrens wird die durch das Sieb geleitete Masse bei einer Temperatur von 20° bis 90°C, vorzugsweise 50° bis 80°C getrocknet. Die Trocknung erfolgt in der gleichen Weise wie die oben im Zusammenhang mit Stufe (c) beschriebene Vortrocknung. Auch hier können übliche Trockner, wie Trockenschränke und Wirbelschichttrockner, eingesetzt werden.

In der letzten Stufe (f) dieses Verfahrens wird schliesslich das so erhaltene Produkt auf eine mittlere Komgrösse von 10 bis 3000 µm, vorzugsweise 200 bis 2000 µm, vermahlen. Für den Vermahlungsvorgang können alle beliebigen geeigneten Mühlen, wie beispielsweise Kugelmühlen und dergleichen, verwendet werden. Geeignete Vorrichtungen sind beispielsweise in Bauer, Frömming, Führer: Pharmazeutische Technologie, G. Fischer Verlag, Stuttgart, Jena, 5. Auflage, 1997, Seite 104-107, beschrieben.

Eine andere Alternative des erfindungsgemässen Verfahrens ist durch folgende Stufen charakterisiert:
(a) Vermischen der pulverförmigen Bestandteile Cellulose, native Stärke und Sprengmittel;
(b) Verdichten durch Kompaktieren oder Brikettieren des Ausgangspulvergemisches, z.B. mit Kompaktierwalzen; und
(c) Granulieren bzw. verkleinerndes Homogenisieren der nach (b) erhaltenen Briketts oder Schülpen mit Stachelwalzen oder einem ähnlichen Gerät oder Vermahlen mit einer geeigneten Mühle auf Korngrößen von 10-3000 µm, vorzugsweise von 200 bis 2000 µm.

Bei dieser Alternative entfallen die Herstellungsschritte der Suspendierung, des Vortrocknens, das Pressen durch Siebe oder Extruder und der Endtrocknung der ersten Alternative. Es verbleiben nur die Schritte Vermischen der beiden Komponenten (a), Kompaktierung des Ausgangspulvergemisches (b), und die nachfolgende Zerkleinerung der kompaktierten Schülpen oder Briketts und das Vermahlen mit geeigneten Mühlen (c).

Die Qualität der Verdichtung hängt von den Kompaktierdrücken und in zweiter Linie auch noch von den Restfeuchtigkeitsgehalten der Mischung ab, die zwischen 3 und 40 Gew.-%, vorzugsweise zwischen 5 und 25 Gew.-% liegen kann. Endprodukte mit den höheren Feuchtigkeitsgehalten müssen evtl. nachgetrocknet werden, bis sie Restfeuchtigkeiten von 1,5-15 Gew.-%, vorzugsweise von 5 - 9 Gew.-% aufweisen. Die Trockenverdichtungs- bzw. Trockengranulierverfahren werden beispielsweise in Bauer, Frömming, Führer: Pharmazeutische Technologie, G. Fischer Verlag, Stuttgart, Jena, 5.Auflage, 1997, Seite 306, 308 und 310, beschrieben.

Die so hergestellten erfindungsgemässen, mit Sprengmitteln coprozessierten Polysaccharidprodukte zeichnen sich durch eine ausserordentlich schnelle und starke Quellung und anschliessenden Zerfall der Tabletten beim Einbringen in Wasser oder wasserhaltige Flüssigkeiten, sowie durch eine sehr gute Tablettierbarkeit aus. Sie können daher als äusserst wirksame Tablettensprengmittel (Zerfallhilfsmittel bzw. Zerfallsbeschleuniger) eingesetzt werden.

Charakteristisch für die erfindungsgemässen, hochwirksamen Sprengmittelgranulate ist der durch die Mischung und Coprozessierung des Sprengmittels mit den Polysacchariden erreichte innige Kontakt der Ausgangsmaterialien. Dieser enge Kontakt der Ausgangsmaterialien soll auch nach dem Einarbeiten von Wirkstoffen (z.B. Arzneistoffe o.a.) und anderen Komponenten in die Tablettenrezeptur erhalten bleiben, denn dieser enge Kontakt ist für die optimale Sprengwirkung entscheidend. Ein weiterer Vorteil dieser intensiv coprozessierten, kombinierten Zerfallsbeschleuniger ist es, dass auch mechanisch feste, stark komprimierte, weniger poröse Tabletten hergestellt werden können.

Unter Verwendung der erfindungsgemässen Produkte können Dispersionstabletten hergestellt werden, die beim Einbringen in Wasser sofort, also in Sekundenschnelle, quellen, zerfallen und sich dispergieren. Je nach Vermahlungsgrad des erfindungsgemässen Produktes lassen sich aus den damit hergestellten Tabletten instantartig gröbere, feine und feinste bis kolloidartige Dispersionen erzeugen. Neben der Verwendbarkeit als Tablettenzerfallsbeschleuniger ist natürlich ein Einsatz als Dispersions- oder Suspensionsstabilisator bei der direkten Herstellung von flüssigen und halbfesten Arzneizubereitungen wie Sirupen, Lotionen, Säften, Gelen, Hydrosolen und Hydrogelen, Salbenzubereitungen und Pasten denkbar und möglich. Die Einsatzmöglichkeiten sind überall möglich, wo schnelle und feinste Dispergierungen erreicht und stabilisiert werden sollen. Die Anwendung ist nicht auf Pharmazeutika und Kosmetika beschränkt. Es drängen sich auch Verwendungen bei nahrungsmitteltechnologischen und technischen Aufgabenstellungen auf. Beispielsweise soll hier die Stabilisierung von Speiseeis oder Softeis, die Verdickung und Dispergierung von Soßen genannt werden, sowie auch Instantzubereitungen, wie Kakao- und andere Mixgetränke, die aus pulver- oder granulatartigen Vorprodukten augenblicklich anrührbar oder dispergierbar sein sollen.

Die Erfindung wird anhand der folgenden Beispiele erläutert.

### Herstellungsbeispiele

1. 300 kg Cellulosepulver mit einer Partikelgrösse < 50 µm werden mit 50 kg unlöslicher Carboxymethylstärke und 150 kg Maisstärke 1 Stunde lang in einem Fassmischer homogen gemischt und anschliessend durch ein Sieb mit 0,5 mm lichter Maschenweite geschlagen. Anschliessend wird diese Mischung über gegenläufige Kompaktierwalzen bei Drücken zwischen 7 und 20 kN/cm kompaktiert, und die entstehenden Schülpen auf eine mittlere Partikelgrösse von 0,2 bis 2,0 mm zerkleinert. Diese verdichteten Granulate werden in Anteilen von 3-10 % zu Tablettenmischungen hinzugemischt, und ergeben auf diese Weise sehr rasch zerfallende Tabletten (je nach Löslichkeit der weiteren in den Tabletten enthaltenen Substanzen 10 bis 60 Sekunden).
2. 500 kg mikrokristalline Cellulose (mittlere Korngrösse 50 bis 90 µm) werden mit 200 kg unlöslicher Carboxymethylcellulose (z.B Nymcel® ZSB 10) gemischt und anschliessend mit 500 I Wasser gleichmässig befeuchtet. In die sich ergebende feuchtplastische Masse werden anschliessend 300 kg Weizenstärke zum Aufsaugen von Feuchtigkeit homogen eingeknetet und durch einen Lochscheiben-Extruder verdichtet. Die hierbei entstandenen Zylindergranulate werden auf eine Restfeuchte von 3-7% getrocknet, und anschliessend auf eine mittlere Partikelgrössen von 0,5 bis 1,0 mm Durchmesser zerkleinert. Diese Granulate dienen als Sprengmittel zur Herstellung von Formulierungen für Tabletten.
3. 340 kg Cellulosepulver (mittlere Partikelgrösse 50 µm), 330 kg vemetztes Polyvinylpyrrolidon (z.B. Sokalan® HP 62) und 330 kg Maisstärke werden homogen gemischt Anschliessend wird diese Mischung über gegenläufige Walzen bei Drücken von 5-20 kN/cm kompaktiert und die hierbei entstandenen Schülpen schliesslich auf Granulatdurchmesser von 0,2 bis 2,0 mm zerkleinert.
4. 600 kg Cellulosepulver mit einer mittleren Korngrösse von 70 µm und 200 kg vemetztes Polyvinylpyrrolidon werden über gegenläufige Brikettierwalzen bei Drücken von 7-15 kN/cm verdichtet, und eine mittlere Korngrösse der hierbei entstandenen Briketts von 0,5 bis 1,0 mm zerkleinert; zuletzt werden 200 kg Kartoffelstärke trocken zu dem Brikettgranulat hinzugemischt. Es entsteht eine Granulatzubereitung mit grosser Sprengkraft, die als Sprengmittelzusatz in 10 bis 20%-Mengen bei der Herstellung von Tabletten eingesetzt werden kann.
5. 400 kg Cellulosepulver (mittlere Partikelgrösse 50 µm), 200 kg vernetztes Polyvinylpyrrolidon und 400 kg Maisstärke werden etwa eine Stunde lang in einem geigneten, verschlossenen Mischer gleichmässig vermischt. Anschliessend wird diese Mischung bei Drücken zwischen 7 und 25 kN/cm über Walzenkompaktoren kompaktiert bzw. verdichtet und die entstandenen Schülpen auf eine mittlere Granulatkömung von 1,0 mm zerkleinert.

## Patentansprüche

1. Coprozessiertes Sprengmittelgranulat für in Flüssigkeit aufzulösende Festkörper, enthaltend unlösliches, extrem hydrophiles, stark quellendes Polysaccharid oder synthetisches Polymer, eine, eine Dochtwirkung aufweisende, wassertransportierende fasrige Cellulose, und eine kugelförmige, gekörnte, native Stärke als Sprengmittel mit begrenzter Dochtwirkung, wobei das Sprengmittelgranulat eine gegenüber dem Ausgangspulvergemisch höhere Schüttdichte aufweist.

2. Sprengmittelgranulat nach Anspruch 1, **dadurch gekennzeichnet, dass** es 20 bis 80 Gew.-% Cellulose, 10 bis 40 Gew.-% native Stärke, sowie 10 bis 40 Gew.-% eines in wässrigem Milieu unlösliches, extrem hydrophiles, stark quellendes Polysaccharid oder synthetisches Polymer enthält.

3. Sprengmittelgranulat nach Anspruch 1 oder 2, **dadurch gekennzeichnet, dass** es als unlösliches, extrem hydrophiles, stark quellendes Polysaccharid oder synthetisches Polymer vernetztes Polyvinylpyrrolidon enthält.

4. Sprengmittelgranulat nach Anspruch 1 oder 2, **dadurch gekennzeichnet, dass** es als unlösliches, extrem hydrophiles, stark quellendes Polysaccharid oder synthetisches Polymer niedrig substituierte und/oder vemetzte Stärke, wie z.B. Carboxymethylstärke enthält.

5. Sprengmittelgranulat nach Anspruch 1 oder 2, **dadurch gekennzeichnet, dass** es als unlösliches, extrem hydrophiles, stark quellendes Polysaccharid oder synthetisches Polymer niedrig substituierte und/oder vemetzte Cellulose, wie z.B. Carboxyethylcellulose, enthält.

6. Sprengmittelgranulat nach einem der Ansprüche 1 bis 5, **dadurch gekennzeichnet, dass** es eine mittelere Korngrösse im Bereich von 10 bis 3000 µm, vorzugsweise im Bereich von 200 bis 2000µm, aufweist.

7. Verfahren zur Herstellung eines Sprengmittelgranulat nach einem oder mehreren der Ansprüche 1 bis 6, **dadurch gekennzeichnet, dass** die Coprozessierung durch feuchte oder trockene Aggregierung unter Druck des Ausgangspulvergemisches erfolgt.

8. Verfahren nach Anspruch 7, **dadurch gekennzeichnet, dass** es die folgenden Verarbeitungsstufen umfasst:
a) Suspendieren und gegebenenfalls Verdichten von pulverisierter Cellulose oder von feuchter bzw. nicht getrockneter, nicht verhornter mikrokristalliner Cellulose in einem vorzugsweise heissen, wässrigen Medium zu einer homogenen Dispersion;
b) Vermengen der in Stufe (a) erhaltenen Suspension oder pastenförmigen, vorverdichteten Masse, vorzugsweise nach Abkühlen unter 60°C, mit nativer Stärke sowie dem in Wasser oder wasserhaltiger Flüssigkeit unlöslichen, quervernetzten Polymer;
c) gegebenenfalls erfolgendes Vortrocknen;
d) Verdichtendes Passieren, vorzugsweise Pressen, der in Stufe (b) bzw. (c) erhaltenen und vorverdichteten feuchtplastischen Masse durch ein Sieb, eine Lochscheibe oder einen Extruder,
e) Trockenen der in Stufe (d) erhaltenen aggregierten Masse bei einer Temperatur im Bereich von 20°C bis 90°C; und
f) Vermahlen des derart erhaltenen Produktes auf eine mittlere Korngrösse im Bereich von 10 bis 3000 µm, vorzugsweise im Bereich von 200 bis 2000 µm.

9. Verfahren nach Anspruch 7, **dadurch gekennzeichnet, dass** es die folgenden Verarbeitungsstufen umfasst::
a) Homogenes Vermischen der pulverförmigen Ausgangsstoffe;
b) Verdichten durch Kompaktieren oder Brikettieren des Ausgangspulvergemisches, z.B. mit Kompaktierwalzen oder einer Exzenterpresse; und
c) Granulieren bzw. verkleinerndes Homogenisieren der nach (b) erhaltenen Briketts oder Schülpen auf eine Korngrösse im Bereich von 10 bis 3000 µm, vorzugsweise im Bereich von 200 bis 2000 µm.

## Claims

1. Coprocessed disintegrant granulate for solids which dissolve in liquid, comprising insoluble, extremely hydrophilic, highly swelling polysaccharide or synthetic polymer which has a water-transporting fibrous cellulose having a wick effect, and a spherical granular native starch and disintegrant with limited wick effect, where the disintegrant granulate has a bulk density which is higher than that of the starting powder mixture.

2. Disintegrant granulate according to Claim 1, **characterized in that** it comprises 20 to 80% by weight of cellulose, 10 to 40% by weight of native starch, and 10 to 40% by weight of an extremely hydrophilic, highly swelling polysaccharide or synthetic polymer which is insoluble in an aqueous medium.

3. Disintegrant granulate according to Claim 1 or 2, **characterized in that** it comprises crosslinked polyvinylpyrrolidone as insoluble, extremely hydrophilic, highly swelling polysaccharide or synthetic polymer.

4. Disintegrant granulate according to Claim 1 or 2, **characterized in that** it comprises starch which has a low degree of substitution and/or is crosslinked, such as, for example, carboxymethyl starch, as insoluble, extremely hydrophilic, highly swelling polysaccharide or synthetic polymer.

5. Disintegrant granulate according to Claim 1 or 2, **characterized in that** it comprises cellulose which has a low degree of substitution and/or is crosslinked, such as, for example, carboxyethyl-cellulose, as insoluble, extremely hydrophilic, highly swelling polysaccharide or synthetic polymer.

6. Disintegrant granulate according to one of Claims 1 to 5, **characterized in that** it has an average particle size in the range from 10 to 3 000 µm, preferably in the range from 200 to 2 000 µm.

7. Process for preparing a disintegrant granulate according to one or more of Claims 1 to 6, **characterized in that** the coprocessing takes place by wet or dry aggregation with compression of the starting powder mixture.

8. Process according to Claim 7, **characterized in that** it comprises the following processing stages:
a) suspension and optional compression of pulverized cellulose or of wet or undried, nonkeratinized microcrystalline cellulose in a preferably hot, aqueous medium to give a homogeneous dispersion,
b) blending the suspension or paste-like precompressed mass obtained in stage (a), preferably after cooling to below 60°C, with native starch and the crosslinked polymer insoluble in water or water-containing liquid;
c) optional predrying;
d) compactive conveyance, preferably pressing, of the precompressed wet-plastic mass obtained in stage (b) or (c) through a sieve, a perforated disc or an extruder;
e) drying of the aggregated mass obtained in stage (d) at a temperature in the range from 20°C to 90°C; and
f) grinding of the product obtained in this way to an average particle size in the range from 10 to 3 000 µm, preferably in the range from 200 to 2 000 µm.

9. Process according to Claim 7, **characterized in that** it involves the following processing stages:
a) homogeneous mixing of the pulverulent starting material;
b) compression by compaction or briquetting of the starting powder mixture, e.g. using compacting rolls or an eccentric press; and
c) granulation or reducing homogenization of the briquettes or flakes obtained according to (b) to a particle size in the range from 10 to 3 000 µm, preferably in the range from 200 to 2 000 µm.

## Revendications

1. Granulat d'agents désintégrants co-travaillés pour un corps solide à dissoudre dans un liquide, contenant un polysaccharide ou un polymère synthétique insoluble, extrêmement hydrophile, à gonflement marqué, une cellulose fibreuse, porteuse d'eau, présentant un effet de mèche, et un amidon natif, granuleux sous forme de billes, en tant que désintégrant à effet de mèche limité, le granulat d'agents désintégrants présentant une densité en vrac plus élevée par rapport au mélange de poudre de départ.

2. Granulat d'agents désintégrants selon la revendication 1, **caractérisé en ce qu'**il contient de 20 à 80% en poids de cellulose, de 10 à 40% en poids d'amidon natif ainsi que de 10 à 40% en poids d'un polysaccharide ou d'un polymère synthétique insoluble en milieu aqueux, extrêmement hydrophile, à gonflement marqué.

3. Granulat d'agents désintégrants selon la revendication 1 ou 2, **caractérisé en ce qu'**il contient, en tant que polysaccharide ou que polymère synthétique insoluble, extrêmement hydrophile, à gonflement marqué, de la polyvinylpyrrolidone réticulée.

4. Granulat d'agents désintégrants selon la revendication 1 ou 2, **caractérisé en ce qu'**il contient, en tant que polysaccharide ou que polymère synthétique insoluble, extrêmement hydrophile, à gonflement marqué, un amidon faiblement substitué et/ou réticulé, comme par exemple l'amidon de carboxyméthyle.

5. Granulat d'agents désintégrants selon la revendication 1 ou 2, **caractérisé en ce qu'**il contient, en tant que polysaccharide ou que polymère synthétique insoluble, extrêmement hydrophile, à gonflement marqué, de la cellulose faiblement substituée et/ou réticulée, comme par exemple de la carboxyéthylcellulose.

6. Granulat d'agents désintégrants selon l'une quelconque des revendications 1 à 5, **caractérisé en ce qu'**il présente une granulométrie moyenne dans le domaine de 10 à 3000 µm, de préférence, dans le domaine de 200 à 2000 µm.

7. Procédé de fabrication d'un granulat d'agents désintégrants selon une ou plusieurs des revendications 1 à 6, **caractérisé en ce que** le traitement en parallèle se fait par agrégation humide ou sèche, sous pression, du mélange de poudre de départ.

8. Procédé selon la revendication 7, **caractérisé en ce qu'**il comprend les étapes de mise en oeuvre suivantes, à savoir :
a) la suspension et, le cas échéant, la compression de cellulose pulvérisée ou de cellulose microcristalline humide ou non séchée, non kératinisée dans un milieu aqueux, de préférence chaud, pour former une dispersion homogène ;
b) le mélange de la suspension obtenue dans l'étape (a) ou de la masse pâteuse comprimée au préalable, de préférence, après refroidissement en dessous de 60° C , à un amidon natif ainsi qu'au polymère réticulé, insoluble dans l'eau ou dans le liquide contenant de l'eau ;
c) le cas échéant, le séchage préalable consécutif ;
d) le passage de compression, de préférence, la compression de la masse plastique humide, obtenue dans l'étape (b) ou (c) comprimée au préalable à travers un tamis, un disque à perforations ou une extrudeuse ;
e) le séchage de la masse agglomérée obtenue dans l'étape (d) à une température dans le domaine de 20°C à 90°C ; et
f) la mouture du produit ainsi obtenu à une granulométrie moyenne dans le domaine de 10 à 3000 µm, de préférence, dans le domaine de 200 à 2000 µm.

9. Procédé selon la revendication 7, **caractérisé en ce qu'**il comprend les étapes de mise en oeuvre suivantes, à savoir :
a) le mélange homogène des substances pulvérulentes de départ ;
b) la compression par compactage ou briquetage du mélange pulvérulent de départ, par exemple par des rouleaux de compression ou une presse à excentrique ; et
c) la mise sous forme de granulat, respectivement l'homogénéisation à réduction de la briquette ou de la gale obtenue en (b) à une granulométrie dans le domaine de 10 à 3000 µm, de préférence, dans le domaine de 200 à 2000 µm.
